(19) Europäisches Patentamt
European Patent Office
Office européen des brevets

(11) **EP 0 885 854 B1**

(12) **EUROPÄISCHE PATENTSCHRIFT**

(45) Veröffentlichungstag und Bekanntmachung des Hinweises auf die Patenterteilung:
**06.12.2000   Patentblatt 2000/49**

(51) Int Cl.[7]: **C03C 3/062**, C03C 3/112, A61K 6/06, C03C 4/08

(21) Anmeldenummer: **98110032.4**

(22) Anmeldetag: **02.06.1998**

(54) **Alumofluorosilicatglas**

Aluminofluorosilicate glass

Verre d'aluminofluorosilicate

(84) Benannte Vertragsstaaten:
**CH DE GB LI**

(30) Priorität: **19.06.1997   DE 19726103**

(43) Veröffentlichungstag der Anmeldung:
**23.12.1998   Patentblatt 1998/52**

(73) Patentinhaber: **ERNST MÜHLBAUER KG**
**22547 Hamburg (DE)**

(72) Erfinder:
• **Lück, Rainer, Dr.**
  **25436 Tornesch (DE)**

• **Reif, Dieter, Dr.**
  **98529 Suhl (DE)**
• **Leuner, Barbara, Dr.**
  **98693 Ilmenau (DE)**

(74) Vertreter: **Glawe, Delfs, Moll & Partner**
**Patentanwälte**
**Rothenbaumchaussee 58**
**20148 Hamburg (DE)**

(56) Entgegenhaltungen:
**EP-A- 0 475 239          US-A- 4 814 362**
**US-A- 5 360 770**

**Beschreibung**

Die vorliegende Erfindung betrifft eine Glaszusammensetzung, nämlich ein Alumofluorosilicatglas, das als Bestandteil von Zahnrestaurationsmaterial geeignet ist. Alumofluorosilicatgläser als Bestandteil von Materialien zur Zahnrestauration sind bekannt. Sie finden insbesondere Verwendung in Glasionomerzementen (definiert in ISO 7484) und in polymerisierbaren Zementen, wie sie in EP-A-0 219 058 beschrieben werden und unter der Bezeichnung "Compomer" und "kunststoffverstärkter Glasionomerzement" bekannt sind (Quintessenz 47, 1581 (1996)).

Dentalmaterialien dieser Art, insbesondere Glasionomerzemente, sind häufig transparent für Röntgenstrahlung. Mangels Röntgenkontrast ist dann eine röntgenografische Untersuchung eines mit einem solchen Zement restaurierten Zahns nicht möglich.

Im Stand der Technik ist bereits vorgeschlagen worden, Dentalzementen Röntgenopazität zu verleihen. Eine Methode ist das Beimischen anorganischer röntgenopaker Füllstoffe, die beispielsweise höhere Erdalkalioxide und/oder Selteneerdenoxide enthalten (DE-A 30 39 664, WO-A 86/00021, US-A 4,629,746). Bei Glasinomerzementen ist vorgeschlagen worden, zusätzlich zu der Polyalkenoatkomponente und Glaskomponente ein röntgenopakes, anorganisches Füllmaterial, beispielsweise Strontiumfluorid, zuzugeben (WO-A 88/05651, EP-A 244 959).

Der Stand der Technik hat sich auch mit der Entwicklung von Alumofluorosilicatgläsern befaßt, die selbst Röntgenopazität aufweisen (US-A 4,814,362, US-A 5,360,770, WO-A 93/17653, DE-A 38 04 469, EP-A 475 239).

Ein Dentalmaterial sollte außer einer gewissen Röntgenopazität auch eine gewünschte Transluzenz bei sichtbarem Licht aufweisen. In diesem Zusammenhang ist es von Bedeutung, daß der Brechungsindex bzw. die Brechzahl der verschiedenen Komponenten der Dentalmischung aufeinander abgestimmt sind.

Der Stand der Technik hat sich in der bereits genannten EP-A 475 239 bereits damit befaßt, sowohl die Brechzahl als auch die Röntgenopazität eines Glases einzustellen. Jedoch beschreibt diese Druckschrift einen komplex zusammengesetzten Dentalzement, dessen Brechungsindex nur in engen Grenzen einstellbar ist.

Der vorliegenden Erfindung liegt die Aufgabe zugrunde, ein röntgenopakes Alumofluorosilicatglas zu schaffen, dessen Brechungsindex $n_D$ für sichtbares Licht auf einfache Weise einstellbar ist und so an den Brechungsindex des in der Dentalmischung verwendeten Polymersystems angepaßt werden kann.

Diese Aufgabe wird durch ein Alumofluorosilicatglas gelöst, das durch folgende Merkmale gekennzeichnet ist:

  a) ein Masseverhältnis von Al (berechnet als $Al_2O_3$) zu Si (berechnet als $SiO_2$) von 0,57-1,12;
  b) einen Gesamtgehalt von Mg (berechnet als MgO) und Ba (berechnet als BaO) von 29-36 Masse-%;
  c) ein Masseverhältnis von Mg (berechnet als MgO) zu Ba (berechnet als BaO) von 0,028-0,32;
  d) einen Gehalt von P (berechnet als $P_2O_5$) von 2-10 Masse-%.

Das erfindungsgemäße Glas erhält Röntgenopazität insbesondere durch den Gehalt von Bariumoxid. Im Rahmen der Erfindung bezeichnet der Begriff "röntgenopak" eine Opazität, die mindestens der von Aluminium entspricht. Eine 1 mm dicke Schicht des erfindungsgemäßen Glases weist also mindestens die gleiche Röntgenopazität wie eine 1 mm dicke Aluminiumschicht auf.

Der Brechungsindex $n_D$ kann bei dem erfindungsgemäßen Glas überraschenderweise gezielt eingestellt werden durch die Variation des als $P_2O_5$ berechneten Phosphorgehaltes. Bei dem erfindungsgemäßen Glas hat eine Veränderung der Anteile der übrigen Glasbestandteile demgegenüber nur eine signifikant geringere Auswirkung auf den Brechungsindex des Glases.

Bei dem erfindungsgemäßen Glas läßt sich der Brechungsindex als Funktion des $P_2O_5$-Gehalts durch folgende empirisch abgeleitete Geradengleichung ausdrücken:

$$n_D = -0,0111 \, (P_2O_5 \text{ in Masse-\%}) + 1,584$$

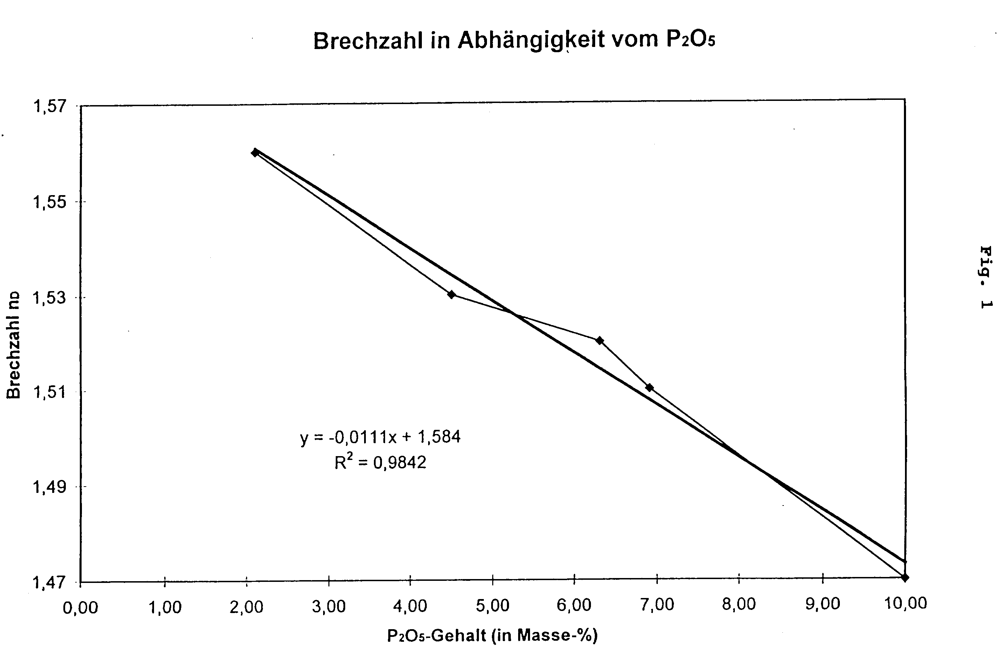

Diese Geradengleichung ermöglicht mit einem Bestimmtheitsmaß von 98% eine Berechnung des Brechungsindexes als Funktion des $P_2O_5$-Gehalts (s. auch Fig. 1).

Bei dem erfindungsgemäßen Glas ist somit die Brechzahl durch den $P_2O_5$-Gehalt steuerbar, die Röntgenopazität kann variiert werden durch die Änderung der Zusammensetzung des Glases im übrigen, insbesondere durch Variation des Bariumoxidgehalts innerhalb des erfindungsgemäß vorgegebenen Rahmens.

Vorteilhafterweise weist das erfindungsgemäße Glas einen Fluoridgehalt von 7-14 Masse-% auf. Bevorzugt ist gleichfalls ein Gehalt von 0,5-5 Masse-% Natrium (berechnet als Dinatriumoxid).

Bevorzugt enthält das Glas folgende Bestandteile (in Masse-%):

-  Al (berechnet als $Al_2O_3$)      20 - 28
-  Si (berechnet als $SiO_2$)      25 - 35

- Ba (berechnet als BaO)     25 - 35
- Mg (berechnet als MgO)     1 - 8
- Na (berechnet als Na$_2$O)     0,5 - 5
- F     7 - 14

Die Aufzählung dieser Bestandteile ist nicht abschließend, andere Bestandteile (beispielsweise weitere Alkali- oder Erdalkalimetalle und/oder Seltenerdmetalle) sowie Verunreinigungen können vorhanden sein.

Die Herstellung eines erfindungsgemäßen Alumofluorosilicatglases erfolgt in bekannter Weise durch Erschmelzen aus den (bevorzugt analysenreinen) Ausgangsstoffen, die die einzusetzenden Metalle beispielsweise als Oxide, Fluoride und/oder Phosphate enthalten können. Nach Erhalt einer homogenen Schmelze wird diese in destilliertem Wasser gefrittet und auf die gewünschte Korngröße zerkleinert, insbesondere gemahlen. Bevorzugt zur Verwendung in Dentalzementen sind Korngrößen mit einer mittleren Korngröße unterhalb von 10 µm, beispielsweise etwa 4 µm, oder auch um 1 µm.

Das erfindungsgemäße Alumofluorosilicatglas kann als Bestandteil eines Dentalmaterials für die prothetische, konservierende und/oder präventive Zahnbehandlung verwendet werden.

Besonders geeignet ist das erfindungsgemäße Glas als Bestandteil eines röntgenopaken, polymerisierbaren Zements. Durch Zugabe des erfindungsgemäßen Alumofluorosilicatglases können polymerisierbare Zemente hergestellt werden, die aufgrund der möglichen Anpassung des Brechungsindexes des Glasbestandteils an den des verwendeten Polymersystems eine gute Transluzenz aufweisen. Die Röntgenopazität dieses Materials kann im Bereich von 200-300% liegen, d.h. eine 1 mm dicke Schicht des polymerisierbaren Zements weist die gleiche Röntgenopazität wie eine 2-3 mm dicke Aluminiumschicht auf.

Das erfindungsgemäße Glas kann ferner zur Herstellung eines Glasionomerzements gemäß ISO 7484 verwendet werden.

In der Zeichnung zeigt Fig. 1 grafisch die Abhängigkeit des Brechungsindex vom P$_2$O$_5$-Gehalt des Glases. Durch die Meßpunkte ist eine empirische Gerade gelegt worden, deren Geradengleichung oben bereits genannt worden ist.

Ausführungsbeispiele der Erfindung werden nachfolgend erläutert.

**Beispiel 1:**

Herstellung erfindungsgemäßer Alumofluorosilicatgläser.

Quarzmehl, Tonerdehydrat, Aluminiumfluorid, Aluminiumphosphat, Kryolith, Bariumfluorid und Magnesiumcarbonat werden in einem Platintiegel bei 1.400°C geschmolzen. Die Mengen der eingesetzten Ausgangsstoffe werden bei jedem Ausführungsbeispiel so gewählt, daß sich Gläser der aus der nachfolgenden Tabelle ersichtlichen Zusammensetzungen ergeben. Es wird erhitzt, bis eine klare, homogene und rückstandsfreie Schmelze vorliegt. Die dünnflüssige Schmelze wird in destilliertem Wasser gefrittet. Das entstandene Glasgranulat wird getrocknet und in dieser Form für die Weiterverarbeitung als Bestandteil von Dentalwerkstoffen zur Verfügung gestellt. Der Brechungsindex n$_D$ der erhaltenen Gläser ist ebenfalls aus der Tabelle ersichtlich.

| Bestandteil (Masse-%) | Glas Nr. 1 | Glas Nr. 2 | Glas Nr. 4 | Glas Nr. 5 | Glas Nr. 6 |
|---|---|---|---|---|---|
| Al$_2$O$_3$ | 20,1 | 26,0 | 26,1 | 27,2 | 23,2 |
| SiO$_2$ | 35,0 | 25,1 | 27,0 | 25,0 | 29,3 |
| BaO | 34,9 | 25,0 | 28,7 | 27,2 | 29,4 |
| P$_2$O$_5$ | 4,5 | 6,9 | 2,1 | 10,0 | 6,3 |
| Na$_2$O | 0,5 | 5,0 | 2,9 | 2,8 | 1,5 |
| MgO | 1,1 | 7,9 | 5,1 | 2,1 | 4,9 |
| F | 6,9 | 7,1 | 14,0 | 9,9 | 9,3 |
| Brechungsindex n$_D$ | 1,53 | 1,51 | 1,56 | 1,47 | 1,52 |

**Beispiel 2:**

Herstellung eines polymerisierbaren Dentalzementes.

Die gemäß obiger Tabelle als Glas Nr. 5 erhaltene Glasfritte wird auf eine mittlere Korngröße von 4 µm gemahlen. 30 g Glaspulver werden mit 7 g Urethandimethacrylat, 5 g Bernsteinsäuremonomethacrylsäureester, 5 g Hexandioldimethacrylat, 1 g Aerosil® R972 (Firma Degussa) sowie 90 mg Campherchinon und 130 mg Dimethylaminobenzolsäure als Starter gemischt.

Aus der erhaltenen Mischung wird ein Prüfkörper mit einer Schichtdicke von 1 mm und einem Durchmesser von 2 cm geformt und dieser Prüfkörper wird 90 s mit einer Dentacolor-XS-Lampe bestrahlt. Das ausgehärtete Material weist eine Röntgenopazität von 200% und eine Opazität bei sichtbarem Licht von 18% auf.

**Patentansprüche**

1. Alumofluorosilicatglas, gekennzeichnet durch folgende Merkmale:

   a) ein Masseverhältnis von Al (berechnet als $Al_2O_3$) zu Si (berechnet als $SiO_2$) von 0,57-1,12;
   b) einen Gesamtgehalt von Mg (berechnet als MgO) und Ba (berechnet als BaO) von 29-36 Masse-%;
   c) ein Masseverhältnis von Mg (berechnet als MgO) zu Ba (berechnet als BaO) von 0,028-0,32;
   d) einen Gehalt von P (berechnet als $P_2O_5$) von 2-10 Masse-%.

2. Alumofluorosilicatglas nach Anspruch 1, dadurch gekennzeichnet, daß der Fluoridgehalt 7-14 Masse-% beträgt.

3. Alumofluorosilicatglas nach einem der Ansprüche 1 oder 2, dadurch gekennzeichnet, daß es 0,5-5 Masse-% Na (berechnet als $Na_2O$) enthält.

4. Alumofluorosilicatglas nach einem der Ansprüche 1 bis 3, dadurch gekennzeichnet, daß es folgende Elemente enthält (in Masse-%):

   - Al (berechnet als $Al_2O_3$)     20 - 28
   - Si (berechnet als $SiO_2$)     25 - 35
   - Ba (berechnet als BaO)     25 - 35
   - Mg (berechnet als MgO)     1 - 8
   - Na (berechnet als $Na_2O$)     0,5 - 5
   - F     7 - 14

5. Verwendung des Alumofluorosilicatglases nach einem der Ansprüche 1-4 zur Herstellung eines Dentalmaterials für die prothetische, konservierende und/oder präventive Zahnbehandlung.

6. Verwendung gemäß Anspruch 5 zur Herstellung eines röntgenopaken polymerisierbaren Zements.

7. Verwendung gemäß Anspruch 5 zur Herstellung eines Glasionomerzements.

**Claims**

1. Aluminofluorosilicate glass, characterized by the following features:

   a) a ratio of Al (calculated as $Al_2O_3$) to Si (calculated as $SiO_2$) of 0.57-1.12 by mass;
   b) a total content of Mg (calculated as MgO) and Ba (calculated as BaO) of 29-36% by mass;
   c) a ratio of Mg (calculated as MgO) to Ba (calculated as BaO) of 0.028-0.32 by mass;
   d) a content of P (calculated as $P_2O_5$) of 2-10% by mass.

2. Aluminofluorosilicate glass according to Claim 1, characterized in that the fluoride content is 7-14% by mass.

3. Aluminofluorosilicate glass according to either of Claims 1 or 2, characterized in that it contains 0.5-5% by mass of Na (calculated as $Na_2O$).

4. Aluminofluorosilicate glass according to any of Claims 1 to 3, characterized in that it contains the following elements (in % by mass):

- Al (calculated as $Al_2O_3$)    20 - 28
- Si (calculated as $SiO_2$)    25 - 35
- Ba (calculated as BaO)    25 - 35
- Mg (calculated as MgO)    1 - 8
- Na (calculated as $Na_2O$)    0.5 - 5
- F    7 - 14

5. Use of the aluminofluorosilicate glass according to any of Claims 1-4 for producing a dental material for prosthetic, conservative and/or preventive dental treatment.

6. Use according to Claim 5 for producing a radiopaque polymerizable cement.

7. Use according to Claim 5 for producing a glass ionomer cement.

**Revendications**

1. Verre au fluorsilicate d'aluminium, caractérisé par les caractéristiques suivantes :

   a) un rapport de masse d'Al (calculé comme $Al_2O_3$) sur Si (calculé comme $SiO_2$) de 0,57 à 1,12 ;

   b) un taux total de Mg (calculé comme MgO) et de Ba (calculé comme BaO) de 29 à 36 % en poids ;

   c) un rapport de masse de Mg (calculé comme MgO) sur Ba (calculé comme BaO) de 0,028 à 0,32 ;

   d) un taux de P (calculé comme $P_2O_5$) de 2 à 10 % en poids.

2. Verre au fluorsilicate d'aluminium selon la revendication 1, caractérisé en ce que le taux de fluorure est de 7 à 14 % en poids.

3. Verre au fluorsilicate d'aluminium selon l'une des revendications 1 ou 2, caractérisé en ce qu'il contient 0,5 à 5 % en poids de Na (calculé comme $Na_2O$).

4. Verre au fluorsilicate d'aluminium selon l'une des revendications 1 à 3, caractérisé en ce qu'il contient les éléments suivants (en % en poids) :

- Al (calculé comme $Al_2O_3$)    20 - 28

- Si (calculé comme $SiO_2$)    25 - 35

- Ba (calculé comme BaO)    25 - 35

- Mg (calculé comme MgO)    1 - 8

- Na (calculé comme $Na_2O$)    0,5 - 5

- F    7-14

5. Utilisation du verre au fluorsilicate d'aluminium selon l'une des revendications 1 à 4 pour la fabrication d'un matériau dentaire destiné à un traitement des dents prothétique, conservateur et/ou préventif.

6. Utilisation selon la revendication 5 pour la fabrication d'un élément polymérisable opaque aux rayons X.

7. Utilisation selon la revendication 5 pour la fabrication d'un ciment d'ionomère de verre.

Fig. 1

Brechzahl in Abhängigkeit vom $P_2O_5$

$y = -0,0111x + 1,584$
$R^2 = 0,9842$

$P_2O_5$-Gehalt (in Masse-%)

Brechzahl nD